# EUROPEAN PATENT SPECIFICATION

(11) **EP 1 171 053 B1**
(45) Date of publication and mention of the grant of the patent: **17.01.2007**
(21) Application number: 00925455.8
(22) Date of filing: 14.04.2000
(51) Int. Cl.: A61C 1/00

(54) **APPARATUS FOR TREATING DENTAL CARIES**
GERÄT ZUR BEHANDLUNG VON ZAHNKARIES
APPAREIL PERMETTANT DE TRAITER LES CARIES DENTAIRES

(30) Priority: 16.04.1999 GB 9908824; 14.01.2000 GB 0000884; 14.01.2000 GB 0000886
(43) Date of publication of application: 16.01.2002
(73) Proprietor: Denfotex Ltd., Crawley Down, West Sussex RH10 4LP (GB)
(72) Inventor: HAMILTON, David, Corbett, Falkirk FK14 7EB (GB); COLLES, Michael, John, Biggar Lanarkshire ML12 6JJ (GB); WILLIAMS, Jill, Ann, Thorpe TW20 8UD (GB); PEARSON, Gavin, John, Reading Berks RG8 8RU (GB); CLEMENTS, David, John, West Sussex RH10 4LP (GB); WILSON, Michael Eastman Dental Institute, London WC1X 8LD (GB)
(74) Representative: Bailey, David Martin
(86) International application number: PCT/GB2000/001433
(87) International publication number: WO 2000/062701

(56) References cited:
- EP-A- 0 637 976
- EP-A- 0 830 850
- EP-A- 0 830 852

## Description

This invention relates to an apparatus for treating dental caries. In general, the apparatus is used in a method which involves killing within or on a tooth bacteria associated with dental caries and then sealing the treated area to prevent re-infection.
There are several situations in which the invention is useful including:-
1. Preventive dentistry: killing bacteria in fissures and on the surface of the tooth prior to sealing and also on the surface of enamel and dentine prior to restoration placement.
2. In conventional treatment as a means of killing residual bacteria in a carious lesion after conventional cavity preparation.
3. A minimally invasive technique with infilling of the lesion with a dentine substitute.

### Background to the Invention

Dental caries is a chronic invasive disease, which can lead to irreparable destruction of tooth tissue. The lesion is created by acid producing bacteria in the plaque. These metabolise carbohydrates and sugars in the plaque and produce acid. The lesion develops first by a demineralisation process of the enamel, the outer covering of the crown of the tooth. The demineralisation produces the porous surfaces, which will eventually permit of migration of bacteria though tubules into the dentine.

Enamel is acellular and once destroyed may not be replaced. Once the advancing demineralisation front reaches the enamel dentine junction, it spreads laterally along the enamel dentine junction leading to further demineralisation on a broad front. The structure of the dentine differs from enamel having an organic matrix, which is calcified. Cell processes run through the dentine initially in the young from pulp to enamel dentine junction. With age this structure becomes more calcified from the outer surface of the dentine inwards.

During the carious process, the dentine is first demineralised, and then this is followed by a phase where the bacteria colonise the denatured dentine. The dentine tubules become dilated and the bacteria can then lie within the lumen of the tubules. They receive nutrients through simple diffusion processes from the outer surface of the enamel. It has been well established that the bacteria, which are anaerobic, will become dormant or die if the supply of nutrient is cut off.

Conventional dental treatment requires that access be gained to the lesion using a bur in a high-speed turbine handpiece. This involves the removal of a substantial amount of sound tooth tissue. As an example, to treat a lesion on the interproximal surface of a tooth part of the both the occlusal and approximal surface are removed in order to access the decay and also to retain the subsequent restoration. The manner of the carious attack results in the enamel being undermined. This means that the enamel will fracture under load and frequently has to be cut back

Currently there are three main commonly used groups of materials available for restoration of the diseased tooth:- dental amalgam, composite resins and glass polyalkenoate cements. The use of gold and ceramic involves a minimum of two visits to the dentist and the costs then become excessive. These materials are generally used where there has been substantial tooth loss and the conventional materials are not able to withstand the clinical loads applied.

Dental amalgam has proved to be the material of choice for the last one hundred years but its use is now being questioned both on environmental, safety and aesthetic grounds. It is however the material which most practitioners find the easiest to use. Dental composites overcome the problem of aesthetics and can withstand moderate degrees of wear but their shrinkage on setting and their demanding technique and technique sensitivity have meant that their time of placement is about one and half that of a dental amalgam. They also require the use of a dentine adhesive to reduce the marginal leakage. These are again technique sensitive. Glass polyalkenoate cements, while being therapeutic because of their fluoride release and uptake, are brittle materials and as yet none of the materials available are suitable for universal use in the oral cavity.

All the materials require that as much as possible of the decay be removed in a manner, which was described by Black in 1890. Identification of infected dentine is reliant on radiographs, probing and visual assessment. At best these are rather crude methods. It is certain that all cavities when completed will retain a number of viable bacteria- Failure to provide a hermetic seal or cut off the supply of nutrient to the bacteria will lead to recurrent decay and the need for eventual replacement of the restoration The average life of an amalgam restoration is of the order of eight years and that of a composite four. This, if extrapolated to a life of a tooth, means that there will be at least five or six replacements of a dental amalgam and double that for a composite resin. Each time the restoration is replaced the cavity is enlarged and there is an increase in the risk of failure as the limit of the restorative material is reached or exceeded. Similarly, as the amount of tooth tissue which is left decreases, then the structural integrity of the tooth is reduced leading to failure of the tooth tissue

WO 93/21992, which is considered to constitute the closest prior art, describes a method of disinfecting or sterilising tissues of the oral cavity or a wound or lesion in the oral cavity by applying a photosensitising compound to the tissues, wound or lesion and irradiating tissues, wound or lesion with laser light at a wavelength absorbed by the photosensitising compound.

EP 0 830 850 Al describes a method of for polymerising a photopolymerisable dental composition with the use of a light-curing apparatus including a laser light source and an optical fibre. The optical fibre is introduced into the dental composition in a dental cavity and the composition is irradiated to cause polymerisation of the composition. Filler in the composition causes scattering of the laser light such that polymerisation is initiated progressively from a portion of the composition adjacent the bottom of the dental cavity towards a surface portion thereof.

### Summary of the Invention

A primary object of the present invention is to provide a kit used in a technique for minimally invasive treatment of dental caries with the minimal removal of tooth tissue, in contrast to current practice. This technique involves the preparation of a small tunnel from the outer surface of the tooth to the site of the carious lesion. The access may be made using a very fine round bur in a high-speed dental handpiece. This will permit the removal of enamel and dentine to produce a narrow tunnel about 400 to 1000 microns, preferably 500 to 800, e.g. about 600 microns in diameter. Instead of a dental drill, a laser-cutting device may be used to cut the tunnel. This will result in a painless and anaesthesia free treatment which can attract those groups of the population who have a phobia about dental treatment and those who currently do not attend for any treatment except for emergencies.

Once access to the lesion has been obtained, the carious dentine will be inoculated with a light photosensitising compound, e.g. a sensitive dye. Examples of suitable photosensitisers are given in European Patent No. 0637976. The bacteria will take up the dye or other photosensitiser and once sensitised, the lesion will be irradiated with light of a specific wavelength. The interaction between the laser and the dye leads to singlet oxygen release and results in the death of the bacteria and this is the basis of photodynamic therapy. Once the bacteria have been killed, the lesion & the demineralised dentine surrounding it, will be infiltrated with a dentine substitute which will be polymerised in situ. Fluoride can be included in this resin composition to provide a therapeutic effect after the photodynamic therapy (PDT) has been completed.

Since the treatment of the carious lesions involves removing the minimum of tooth material, access to the lesions is established by cutting cavities of narrow diameter. Activation of the photosensitiser within the cavity is achieved by using an optical fibre having a small diameter and tip which is less than 1 mm and preferably about 800 microns or less. For very small cavities and fissures, the tip may have a maximum lateral dimension of 400 microns or less, e.g. about 200 microns.

There are a number of types of carious lesions which are advantageously treated. These include accessing the lesion by drilling an access tunnel from the tooth surface to a lesion located beneath the crown. The invention is also applicable to the treatment of root caries, fissure sealing, in advanced restorative work and in treating recurrent caries beneath a restoration or artificial crown.

The present invention provides a kit of parts for treating dental carious lesions which comprises: (a) a flowable photosensitiser comprising a photosensitive dye; (b) an optical fibre which is shaped and dimensioned to enter a dental cavity or recess in which the lesion is located, said fibre being connectable proximally to laser light generating means for generating laser light at a wavelength which is strongly absorbed by the photosensitiser and said fibre having a distal portion which is free from an internally light-reflecting layer and is shaped to spread emitted light around the walls of the recess or cavity; and (c) a sealant or filling composition which is introducible into the cavity or recess and after curing forms an air-tight seal; the photosensitive dye being effective to interact with the laser light to kill bacteria in the carious lesion.

The bacteria associated with dental caries can be killed using a photosensitiser and an activation system. The concentration of the light sensitive dye or other photosensitiser and the sensitisation time are well within the clinical requirements of the practising dentist

The generalised effects of photodynamic therapy have been shown to be limited to the internal structure of the tooth tissue and there appears to be no effect on the cells within the dental pulp.

The effects of attenuation of the light source during its passage through demineralised and partly demineralised dentine has been quantified. It has been found that the photosensitiser will penetrate demineralised dentine and the laser light will pass through a significant thickness of dentine. These findings will permit the dentist to limit the amount of dentine which is removed, compared with conventional practice.

A range of resins with suitable properties for inoculating down a narrow bore hole has been identified and their therapeutic effects may be enhanced by the addition of fluoride. For sealing and filling the narrower cavities envisaged in the practice of this invention, sealant compositions have been devised which are of low viscosity and are capable of flowing into narrow cavities and fissures. Such sealing compositions comprise mixtures of tetrahydrofurfuryl methacrylate (THFMA) (or a derivative thereof), with one or more mono and difunctional acrylates and/or methacrylates. The sealant composition should be fluid enough when syringed into the cavity or recess after the photosensitisation treatment. A typical cavity may have a diameter of about 500 to 1000 microns. Typical viscosities are 0.6 to 1350 centipoise. Preferred sealant compositions comprise a mixture of at least one resin containing on average at least two acrylate or methacrylate groups and a copolymerisable methacrylate or acrylate monomer which has a low viscosity compared with the resin. Preferably, the methacrylate monomer is a heterocyclic dimethacrylate, e.g. THFMA. The resin is preferably a urethane dimethacrylate or a glycidyl dimethacrylate, such as an epoxy dimethacrylate. Such compositions are described in more detail in our co-pending British patent application No. 0000886.2 (Our ref: DFX Case C) filed concurrently herewith.

These resins have been tested for biological acceptability and has been demonstrated to have a biocompatibility similar to Zinc oxide/eugenol, the standard for biocompatibility in dentistry ISO [TR7405]. Details of suitable resins are also given hereinafter.

After introducing the photosensitising agent into the narrow bore hole in the tooth, the agent is activated using light delivered by an optical fibre from a laser at an appropriate wavelength which is absorbed strongly by the photosensitiser. The wavelength employed depends on the absorption spectra of the photosensitiser. Toluidine blue O (ortho-toluidine) is preferably employed as the photosensitiser and has an absorption maximum in the range of 600 to 700 nm, more specifically at 630 to 640 nm. Semiconductor lasers, gallium/arsenide and helium/neon lasers may be used. The laser light may be continuous or pulsed. If the light is pulsed, preferably there is a short interval between the pulses, e.g. a fraction of a second to one second, preferably about ½ second. It has been found to be important to spread the laser light uniformly within the bore hole rather than focus it on a small target area. One way of achieving this is to provide an optical fibre which terminates in an isotropic tip, e.g. a generally spherical tip. A method of forming such a tip is described in US Patent No. 5,073,402.

Essentially, the light-spreading distal tip may be conveniently formed by moulding or casting a curable light transmissive composition on the end of the optical fibre. A spherical tip may be formed by dipping the optical fibre into a polymerisable composition and curing the adherent droplet, while supporting the droplet in a non-miscible liquid. Curing may be effected by passing light of the appropriate wavelengths for curing along the optical fibre. Suitable polymerisable compositions include light curable acrylate and methacrylate compositions, including those described below as suitable sealant materials. It may be desirable to include a light-scattering material within the polymerisable material to increase the uniformity of irradiation of the root canal. However, the cured tip will be transparent or translucent to light of the wavelength selected for sensitising the dye.

Other shaped tips maybe formed by moulding or casting the desired shape onto the end of the optical fibre.

An isotropic light-emitting tip may also be formed by removing the internally reflective outer layer of the optical fibre over a short distance from the distal end, or by restricting the outer layer so that it is not applied to the distal end.

The photosensitising agent may be delivered in a unidose system. The dentine substitute will act in a similar way to the currently available dentine adhesives which set out to infiltrate dentine which has been partly demineralised using acid demineralising agents. The dentine substitute will infiltrate the demineralised dentine and provide an hermetic seal. A secondary role is the strengthening of this layer producing a dentine type substitute which has properties akin to dentine and which will support a small restoration sealing the access cavity.

### The photosensitiser

The photosensitising compound is used for the disinfection of the tooth surface and internal structure by placement using a liquid or gel or varnish in contact with the lesion and irradiating the tissue with light of an appropriate wavelength that will be absorbed by the photosensitiser. The liquid, gel or varnish are generally aqueous. A gel may be formed by including a gelling agent in the aqueous solution of the photosensitiser. Suitable gelling agents include hydrophilic polymers such as cellulose derivatives and polyvinyl pyrrolidone. Alternatively, thickening agents such as colloidal silica (e.g. Cabasil) may be added, e.g. in amounts of up to 10% by weight. Gels are particularly useful in pre-treatment prior to fissure sealing or where the cavity is shallow.

The photosensitiser and laser combination may be applied to:-
A) Disinfection or sterilisation of the drilled out carious lesion.
B) Destruction of carious microbes in or on the tooth structure in order to treat or prevent caries.

Photosensitising agents used in this invention are generally non-toxic to the target microbes in the concentrations envisaged and particularly to the surrounding tissue. However there is no requirement that the photosensitiser should not be toxic to the microbes. Since the exposure time are short it may be acceptable to use compounds which have some slight toxicity to the tissue.

It is preferred that the photosensitisers used will be capable of absorption in the red end of the visible spectrum or at longer wavelength as these wavelengths will have greater penetrating powers in the tissue surrounding the lesion.

The preferred photoinitiators are those effective against Gram Negative bacteria associated with dental caries. These are the dyes. Of these the currently preferred is toluidine blue O. Alternatively aluminium disulphonated phthalocyanine chloride, methylene blue, azure blue chloride may be used. While the dye may be non specific it can be made specific to the microbes within the carious lesion.

The concentration of photo-initiator and laser power are matched to provide maximum penetration of tissue and kill rates
The concentrations of dye range from 0.00001% to 0.1%. The preferred concentration is 0.001 to 0.01 %.

The preferred laser irradiation time of the photosensitiser is between 10 seconds and 90 seconds and the preferred exposure time is 20 to 60 seconds, especially 20 to 40 seconds.

The laser power is preferably between 20 and 600 mW, e.g. 40 to 200 mW, the most preferred being 40 to 150 mW.

The photosensitiser solution concentration may be influenced by any extrinsic fluid and concentration may increased to compensate for this.

In order to improve or increase the rate of access to bacterially infected carious tooth tissue and to enable the photosensitising agents to have maximum effect, the agent may be used in conjunction with, prior to or subsequent to, the photosensitising solution. These may be:-
- Acids to produce a solution pH of 4.5 or above, e.g. 4.5 to 5
- Acids to penetrate and remove organic/inorganic debris
- Wetting agents such as HEMA [hydroxyethyl methacrylate] and glutaraldehyde
- Demineralising agents such as chelating agents of the type EDTA disodium.

Such materials may be citric acid, polyalkenoic and polyphosphonic acid, phosphoric acid, EDTA and HEMA or other such acids as are known for use in this technique. EDTA and citric acid have an adverse effect on the bacterial kill rate if used simultaneously with the photosensitising treatment. Thus, in the case of these additives, they should be used prior to or after treatment with the photosensitiser and the treated area flushed with a wash liquid prior to treatment with the photosensitiser. Phosphoric acid, particularly when buffered to a pH of 4.5 or above appears to have a beneficial effect on bacterial kill rates when used at the same time as the photosensitisation treatment.

A further addition to the photosensitising phase is the addition of remineralising solution to be applied with or subsequent to the application of the dye.

Suitable agents are described by Causton BE and Newell Johnson NW, 1982, BDJ:152, 9-11, Levine RS, Beech DR and Garton B, 1977, BDJ, 143, 275-277 or Pearse and Nelson Caries Research 1988 22,362-370.

It is important that these agents do not interfere with the photosensitising process. In particular the use of free radical and singlet oxygen scavenging materials should be avoided as adjuncts.

The photosensitiser may be delivered by a syringe, for example, by a unit dose device through which the fibre optic probe may be passed, e.g. as described below.

After the bacteria has been killed, the bore hole in the tooth is sealed by introducing a resin as a Dentine Substitute which will infiltrate the remaining dentine structure infilling the porous structure. The resin filling material can be a syringeable resin with low viscosity which may be polymerised using light or by auto-polymerisation. Additionally the resin may release fluoride. If the resin is light-polymerised, light of a suitable wavelength to cure the resin can be conducted into the hole using the same optical fibre. The purpose of the sealant is to exclude air from the treated area, e.g. cavity or recess.

Possible resins which may be used to seal the hole in the tooth include:-

Those as in:-
PCT/GB92102128
PCT/GB98/00072
US 5,172,763
US 5,063,257
EP 0356868
GB 2107 341
US 5,520,725
US 1,428,165
WO 97/00065
UK 1488 403
US 4,267,097
US 4,001,483

The hermetic seal after bacterial killing is an integral part of the non invasive technique since it is by these means that the bacterial re-infection is minimised. This may be achieved using existing dental materials.

The preferred materials may have a viscosity, which may be varied to suit the application. Preferred viscosities are from 0.33 to 1340 centipoise. Where it is used as a dentine substitute, the viscosity is similar to that of water and has mechanical properties such as flexural strength after polymerisation in the range 80 to 170 Mpa. Shrinkage during polymerisation will be in the range of 0.5 to 4.5% by volume. The dentine substitute is made of a blend of resins, which will provide a range of viscosities to suit the intended application.

These may be di-methacrylates or methacrylates as set out in the patents mentioned above. The preferred formulation is a mixture of urethane dimethacrylate (UDMA), bisphenol-A-glycidyl dimethacrylate (BisGMA) and tetrahydrofurfuryl methacrylate (THFMA). These may be in various proportions, the preferred composition being THFMA 50% UDMA 33% and BisGMA 17%.

The material may be polymerised chemically or by the application of light of a particular wavelength. Sealant materials based on light-curable acrylates or methacrylates are commonly cured with light having a wavelength of about 450-470nm.

Cold cure initiator systems which do not require the addition of external energy (heat or light) are materials such as benzoyl peroxide as an initiator and N,N-dimethyl-p-toluidine as an activator.

Preferred light activation systems are those including camphorquinone and an amine. Other activation systems may also be used.

The initiators should be present in an appropriate amount to provide an adequate level of conversion and adequate rate of conversion. They are usually present in amounts between 0.1% and 12% of the weight of the monomer mixture. Preferred values are between 0.5 and 5% by weight of the monomer mixture.

Various additives may optionally be included in the mixture such as antioxidants, stabilisers using UV inhibitors and polymerisation inhibitors, pigments and therapeutic agents such as antibiotics, corticosteroids and other medicinal agents such as metal ions.

The following illustrates the conditions required to effect sterilisation of bacteria associated with dental caries.

### Stage 1

Lethal photosensitisation of Streptococcus mutans in a saline suspension.

Experiments have been carried out using a range of concentrations of Toluidene blue O (TBO). These concentrations have been inoculated into a saline suspension of Streptococcus mutans and then irradiated using the laser equipment shown in the accompanying drawings. A range of power densities and exposure times were evaluated. Full details of exposure times and laser power densities and dye concentrations assessed are set out in Table 1.

**TABLE 1**

| **Dye Concentration [µg/ml]** | **Dye/Bacteria Suspension Volume [µL]** | **Laser Power [mW]** | **Exposure Time [sec]** |
|---|---|---|---|
| 50 | 200 | 80 | 30 |
| 50 | 200 | 48 | 30 |
| 50 | 200 | 15 | 30 |
| 50 | 200 | 80 | 60 |
| 50 | 200 | 48 | 60 |
| 50 | 200 | 15 | 60 |
| 50 | 200 | 15 | 90 |
| 20 | 200 | 80 | 30 |
| 20 | 200 | 15 | 30 |
| 20 | 200 | 80 | 60 |
| 20 | 200 | 15 | 90 |
| 20 | 200 | 15 | 60 |
| 20 | 50 | 40 | 10 |
| 20 | 50 | 40 | 20 |
| 20 | 50 | 40 | 30 |
| 5 | 50 | 40 | 20 |
| 5 | 50 | 40 | 50 |
| 5 | 50 | 40 | 80 |

Dye solutions were made up freshly on each occasion using distilled water. Initially, 100 µl of a saline suspension of Streptococcus mutans was added to a micro well of a microtitre plate. These were derived from Streptococcus mutans NCTC 10449 and suspension was made up to give a concentration of bacteria ranging between 10⁸ to 10⁹ colony forming units (cfu). [These had an optical density of approximately 0.10]. This solution was then gently agitated for the remainder of the experiment. To this was added either 100µl of TBO or saline. Saline acted as a control. 30 seconds after the addition of the dye, the isotropic probe was immersed in suspension and irradiation carried out at the range of exposure time/power density combinations indicated in Table 1.

Aluminium foil was wrapped around the base of each well on the microtitre plate to prevent laser irradiation affecting wells adjacent to that being treated.

After irradiation, the number of survivors in each well was determined by viable counting on tryptone soya agar. 100µl of liquid from the micro well was removed and diluted progressively tenfold. The diluted suspension was then cultured on the tryptone soya agar plate for 24 hours. Plates were then selected with densities of colony forming units of between 50 - 300 cfu and these were examined for surviving bacteria over the whole of the sample on the plate.

### Results

A range of kill levels was achieved with the different combinations of laser power exposure time and dye concentration. This shows that the kill levels required may be achieved at certain laser power/exposure time/dye concentrations. There is a range of combinations, which may be appropriate for use clinically.

### Phase 1:

An exposure time of thirty seconds was arbitrarily established as the baseline value to be examined. The laser parameters were defined by the outputs of the machine and 80 and 15mw selected as the outer limits for initial evaluation. Dye concentrations were fixed at values that had previously been established for penetration of dentine.

The results of the four stages are set out below.

### Exposure Time 30 Seconds

Stage 1: Maximum dye concentration and Maximum laser power

| Dye Concentration [µg/mL] | Laser Power [mW] | Initial CFU | % Bacterial kill Mean | |
|---|---|---|---|---|
| 50 | 80 | E9-E10 | 93.11 | 89.71 |
| | | | 86.32 | |

This indicated that there were very substantial kills but total kills were not on the 99.9% value at this particular exposure time.

### Stage 2: Minimum dye concentration and Minimum laser power

| Dye Concentration [µg/mL] | Laser Power [mW] | Initial CFU | % Bacterial kill Mean | |
|---|---|---|---|---|
| 20 | 15 | 1.1E9 | 0 | 20.0 |
| | | | 40.0 | |

This showed that the minimum output from laser and low dye concentration did not produce adequate kills.

### Stage 3: Minimum dye concentration and Maximum laser power

| Dye Concentration [µg/mL] | Laser Power [mW] | Initial CFU | % Bacterial kill Mean | |
|---|---|---|---|---|
| 20 | 80 | E9-E10 | 99.99 | 99.99 |
| | | | 99.99 | |

This demonstrated that the kill levels, which were required, were achievable at this laser power and dye concentration

### Stage 4: Maximum dye concentration and Minimum laser power

| Dye Concentration [µg/mL] | Laser Power [mW] | Initial CFU | % Bacterial kill Mean | |
|---|---|---|---|---|
| 50 | 15 | 2.1E10 | 0 | 7.05 |
| | | 2.8E9 | 14.1 | |

This shows that maximum dye concentration and minimum laser power produced inadequate kills.

### Conclusions

From this is became apparent that successful kills were obtainable with the minimum dye concentration and maximum laser output. Increasing the dye concentration at that laser power reduced the kill rate. From this it was deduced that the maximum dye concentration was in excess of that required and that better results were achievable at lower concentrations. This was confirmed by the stages two and four where although the kill rates were not very high, the higher dye concentrations produced lower kill rates.

Further work was then carried out using different exposure time and laser energy densities in an attempt to establish the range of combinations, which would prove to be effective. For clarity, the results have been reported in sequence for increasing exposure time.

### Exposure time : 10 sec

| Dye Concentration [µg/mL] | Laser Power [mW] | Initial CFU | % Bacterial kill Mean | |
|---|---|---|---|---|
| 20 | 40 | 2.6E7 | 20.23 | 36.34 |
| | | | 52.45 | |

This was regarded as unsuitable for use. It was not repeated with a lower dye concentration as the increase in kill rates expected was considered to be insufficient to achieve and the exposure time was increased.

### Exposure Time: 20 sec

| Dye Concentration [µg/mL] | Laser Power [mW] | Initial CFU | % Bacterial kill Mean | |
|---|---|---|---|---|
| 5 | 40 | 1.7E7 | 0 | 30.1 |
| | | 1.1E8 | 60.3 | |
| 20 | 40 | 3.0E7 | 79.36 | 81.1 |
| | | 1.8E7 | 82.90 | |

The exposure time of 20 seconds produced kill rates which were substantially higher. At the 20 µg/mL dye concentration tested here 80% kill levels were obtained.

### Exposure Time : 50 sec

| Dye Concentration [µg/mL] | Laser Power [mW] | Initial CFU | % Bacterial kill Mean | |
|---|---|---|---|---|
| 5 | 40 | 7.1E7 | 84.43 | 87.14 |
| | | 1.3E8 | 89.85 | |

The exposure time was extended to 50 seconds with a low dye concentration and low laser power. The kill rates while not being total were extensive. This indicated that the lower dye concentration [5µg/ml] is probably the bottom level that is applicable. It would require a high laser output and longer exposure time for total killing to be achieved.

### Exposure Time : 60 sec

| Dye Concentration [µg/mL] | Laser Power [mW] | Initial CFU | % Bacterial kill Mean | |
|---|---|---|---|---|
| 20 | 15 | 1.2E9 | 86.32 | 52.29 |
| | | 1.1E9 | 18.26 | |
| 20 | 80 | E9-E10 | 99.99 | 99.99 |
| | | | 99.99 | |
| 50 | 15 | 1.3E9 | 12.0 | 12.16 |
| | | 2.8E9 | 12.32 | |
| 50 | 48 | 2.3E10 | 99.76 | 99.78 |
| | | 1.3E9 | 99.8 | |
| 50 | 80 | E9-E10 | 99.99 | 99.99 |
| | | | 99.99 | |

When the exposure time was extended to 60 seconds with a range of laser powers and dye concentrations, it became apparent that kill rates were high for a range of dye concentrations and laser power outputs. It was apparent that the minimum laser power that was successful in achieving kill rates in the region of 10⁹ was 45mW at this time interval.

### Phase 2: Lethal photosensitisation of Strep mutans in a collagen matrix

This section of the study was designed to repeat phase 1 study using a collagen matrix to determine the effect of collagen on light/dye penetration using laser exposure times determined initially. The outcome was to demonstrate that in a collagen matrix kill rates of 10⁸ to 10¹⁰ could be achieved.

### Method

The method is modified from that described previously in Caries Research [1995] 29:192-197. The dye solution selected for use was an aqueous solution with a pH adjusted to 5. No additives were included in this study. Additives were not included at this stage as the work in bacterial suspension showed that additives were reducing the effectiveness of the PDT process. This is an area that will be evaluated further in the next phase.

1009µL of treated rat tail collagen and 50µL of a Strep mutans suspension were mixed together and the solution partly dried overnight to produce a plug with a jelly like consistency. 50µL of TBO [the photosensitiser] was then injected into the centre of the plug using a micropipette. It was noted that the dye solution tended to well up around the sides of the micropipette tip and formed a puddle on the top of the plug. This led to a rather uneven distribution of the dye. Currently, there does not appear to be a way of avoiding this. this is one of the limitations of the experimental method. This inoculation of the dye effectively reduces the dye concentration by a factor of four. [Addition of 40µl/mL to the 150µl/mL collagen/Strep mutans mixture produces this dilution} this is equivalent to an overall concentration of 10µL.

It was noted that the dye did not penetrate into the collagen matrix very quickly. For this reason, a number of contact times were evaluated to find the most appropriate time. Too short a time would mean that the bacteria within the plug might not come into contact with the dye. This would mean a reduction in the number of bacterial kills. Too long an exposure to the dye would lead to "dark kills" where the sensitiser alone had an effect. In order to evaluate this effect, three contact times were investigated, 30 sec, 3 minutes and one hour.

Three irradiation times were used 30, 60 and 180 sec. The laser powers used were 80mW and 60 mW.

Following irradiation, the collagen plug was treated with 50ml of 0.5mg/ml type 1A collagenase for three hours to liberate the survivors and enable the enumeration by viable counting as described above in stage 1.

To evaluate the poor penetration of the sensitiser into the plug, a technique of shredding the collagen plug to make it porous was investigated. This was considered to have a greater similarity to carious dentine than the solid collagen plug.

### Results

The preliminary contact time of 60 minutes was used to evaluate the permeation of the dye through the dense collagen plug.

Contact time of 30 seconds produced good kills with the dye/laser combinations, the dye along having no effect on the bacteria. The results of the different power densities, irradiation times and contact time are set out below, together with the kill rates achieved and percentage kills. The numbers highlighted indicate those achieving the milestone.

### Collagen Plug

### Irradiation Time: 30 seconds. Dye concentration: 10µg/mL

| Contact Time Dye/bacteria [min] | Laser power [mW] | Number killed | % killed | Mean % kill |
|---|---|---|---|---|
| 0.5 | 80 | 2.07E9 | 77.2 | 78.7 |
| | | 1.28E9 | 80.2 | |
| 3 | 80 | 1.36E9 | 64.8 | 75.2 |
| | | 2.95E9 | 85.6 | |
| 60 | 80 | 3.28E8 | 100 | 100 |
| 0.5 | 60 | 1.12E9 | 51.9 | 61.8 |
| | | 2.21E9 | 71.6 | |

### Irradiation Time: 60 seconds. Dye concentration: 10µg/mL

| Contact Time Dye/bacteria [min] | Laser power [mW] | Number killed | % killed | Mean % kill |
|---|---|---|---|---|
| 0.5 | 80 | 2.72E9 | 85.4 | 42.7 |
| | | 0 | 0 | |
| 3 | 80 | 4.85E9 | 94.5 | 92.6 |
| | | 4.29E9 | 90.7 | |
| 60 | 80 | 2.20E9 | 100 | 100 |
| 0.5 | 60 | 1.12E9 | 72.9 | 73.8 |
| | | 2.49 | 74.7 | |

### Irradiation Time: 180 seconds. Dye concentration: 10µg/mL

| Contact Time Dye/bacteria [min] | Laser power [mW] | Number killed | % killed | Mean % kill |
|---|---|---|---|---|
| 0.5 | 80 | 2.96E9 | 91.0 | 51.9 |
| | | 0 | 12.7 | |
| 3 | 80 | 2.23E9 | 91.02 | 93.2 |
| | | 5.7E9 | 95.32 | |
| 60 | 80 | 3.08E8 | 100 | 100 |
| 0.5 | 60 | 1.89E9 | 85.9 | 79.1 |
| | | 2.21E9 | 72.36 | |

It is clear that kill levels of the order of 10⁹ are achievable at both 60 and 80mW with exposure time in excess of 30 seconds. In terms of percentage kills, these increase with the contact time with the bacteria. This demonstrates the importance of ensuring that the dye comes into contact with the bacteria. The collagen plug represents a severe test, as it is not porous and dye/bacteria contact depends on the rate of diffusion through the solid mass of the plug.

In summary, dye contact/dwell times of between 30 seconds and 3 minutes have been found to be satisfactory. Laser irradiation times of between 30 and 180 seconds are generally preferred.

In order to simulate a carious lesion more closely, the collagen matrix was shredded. This provides a porous structure with the bacteria still bound within the collagen. This is similar to a carious lesion where the dentine is porous. The results of this study are set out below.

### Shredded Collagen

| Contact Time Dye/bacteria [min] | Laser power [mW] | Irradiate Time | Number killed | % killed | Mean% kill |
|---|---|---|---|---|---|
| 3 | 80 | 30 | 3.08E9 | 91.1 | 91.05 |
| | | | 3.26E9 | 91.0 | |
| 3 | 80 | 60 | 5.46E9 | 91.3 | 90.8 |
| | | | 3.66E9 | 90.4 | |
| 3 | 80 | 180 | 6.84E9 | 95.73 | 92.1 |
| | | | 1.97E9 | 87.42 | |

This demonstrates that a high kill rate may be achieved in this medium with a short dye bacteria contact time and short laser exposure times.

### Conclusions

1) It can be seen that kill levels of 10⁹ of Strep mutans are achievable in both bacterial suspension and collagen matrix using TBO and the laser described below.
2) The collagen matrix study has demonstrated the importance of contact time between dye and bacteria.

Thus, it may be desirable to maintain the photosensitiser in contact with the lesion for an extended period, e.g. up to about 5 minutes or more, preferably 30 seconds to 3 minutes, before treatment with the laser. During this period, the lesion may be flushed with a solution of the photosensitiser so that the bacteria is contacted with a stream of fresh photosensitised solution.

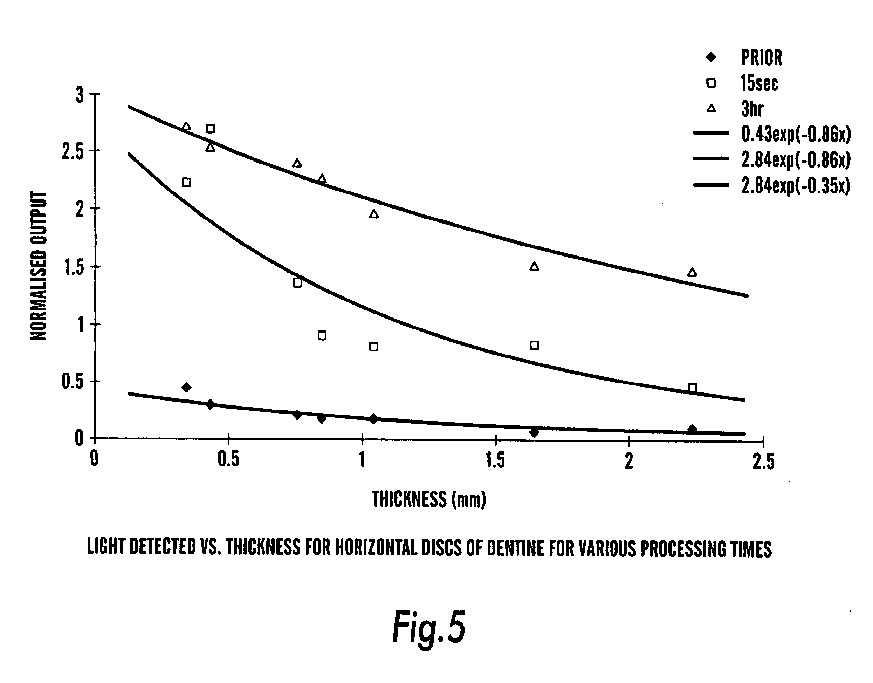

Experiments with slices of dentine cut from extracted human teeth have shown that it is desirable to pretreat a tooth cavity or recess with a demineralisation solution, e.g. of EDTA prior to the photosensitisation treatment. Even a short pre-treatment with EDTA, e.g. as a 0.1 molar aqueous solution, substantially increases the distance through which the laser light can pass. Even pre-treatment of the dentine with 0.1 molar EDTA or other demineralisation solution for as little as 15 seconds increased the depth of light transmission and dye penetration significantly. This is an important finding and enables the dentist to be confident that bacteria has been killed in crevices extending into the dentine of a treated tooth. The effect of demineralisation treatment on the light transmission and dye absorption is shown graphically in Figure 5. The effect of the demineralisation additive appears to be self-limiting in that the maximum demineralised area extends essentially only to the boundary of the dentine affected by the carious lesion.

The accompanying drawings illustrate one manner of carrying out a method of using the invention.
Figure 1 is a schematic cross-section through a tooth with an optical fibre in place in a hole drilled into a decayed internal portion of the tooth;
Figure 1(a) is a plan view of the tooth shown in Figure 1;
Figure 2(a) is a schematic sectional view of a dental handpiece fitted with an optic fibre tip and a tube for introducing photosensitiser into the cavity;
Figure 3 is a perspective view of the laser housing connected to a dental handpiece;
Figure 4 is a view in the direction of arrow X in Figure 3; and
Figure 5 is a graph showing the effect on increasing the light transmission of carious dentine by demineralisation with EDTA.

A tooth (1) is first drilled with a fine dental drill or with a laser to form a narrow tunnel (2) to a decayed area (3). After completing the tunnel, a flexible optical fibre (4) is introduced into the hole until the tip (5) is located close to the closed end of the tunnel The fibre is formed with a generally spherical transparent tip to ensure that light passing down the fibre (4) and emerging at the tip is scattered generally uniformly around the cavity (3).

Photosensitiser comprising an aqueous solution of toluidine blue having a concentration of 50µg/ml, is injected into the tunnel, e.g. using a syringe. After allowing a contact time of about 1 minute, the photosensitiser is activated by connecting the proximal end of the optical fibre to a laser source and the photosensitiser in the cavity (3) irradiated for 60 seconds at a laser power level of 60 mW and wavelength of 640 nm. The interior of the cavity (3) was substantially completely sterilised by this treatment and the tunnel was then filled with a fluid, curable resin, e.g.. using a syringe. The composition employed was a mixture of tetrahydrofurfuryl methacrylate (THFMA), 50 weight percent, urethane dimethacrylate (UDMA) 33 weight percent and Bisphenol-A-glycidyl dimethacrylate (BisGMA) 17 weight percent. This resin is light-curable and is most effectively cured at a wavelength of about 450 to 470 nm. The resin may be cured by directing laser light using an optical fibre having a light spreading tip. The fight may be generated by LED's centred at 470 nm or 150 or 200 mW with an exposure time of between 5 and 30 seconds. The same optical fibre can be used to apply light irradiation to the resin as for carrying out the sterilisation step. However, it is necessary to use a different laser source because of the different wavelengths employed. Once curing is complete, the optical fibre may be cut off, e.g. with a rotary saw blade or simply snapped off and the upper surface smoothed over.

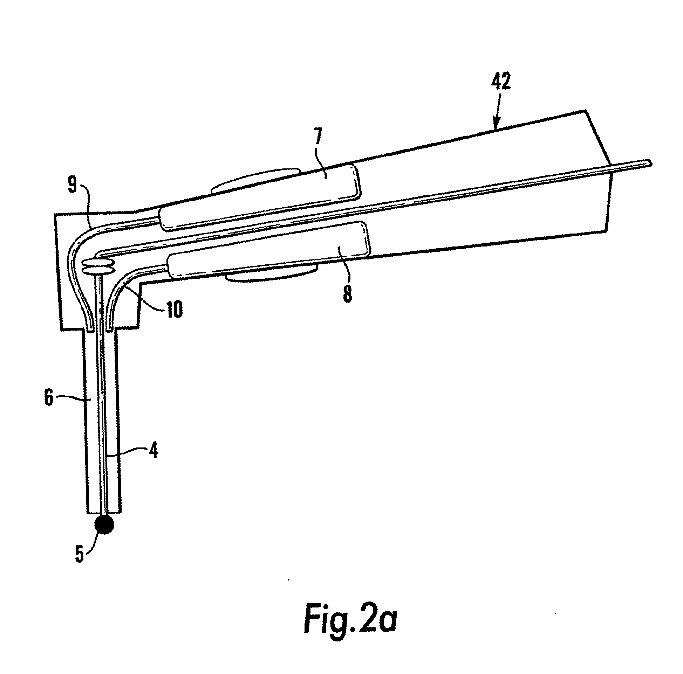

Figure 2a shows a modification of the optical fibre which incorporates a catheter having reservoirs of photosensitiser and resin. In this arrangement, the optical fibre (4) is threaded through a catheter (6). At a point beyond the maximum length which would be introduced into a tooth, two reservoirs (7) and (8) are provided. Reservoir (7) contains photosensitiser and reservoir (8) contains uncured resin. In order to introduce photosensitiser into the cavity, the dentist compresses the reservoir (7), which ruptures a membrane (10) and allows photosensitiser to flow between the optical fibre and the catheter and out of the distal end of the catheter. Once the sterilisation step has been completed, reservoir (8) is squeezed. This causes resin to flow through the gap between the optical fibre and the catheter and displace the photosensitiser to fill the tunneL The resin is then cured as described above and the optical fibre and catheter can then be cut off and the treated dental caries area sealed.

Figure 2(a) shows a developed form of the system. The handpiece (42) has a 'plug in' optic fibre (4) having an isotropic tip (5). The fibre is received as a snap fit in the handpiece (42) and is optically connected through the handpiece to a laser source in the console (41). Surrounding the optic fibre is a hollow tube (6). Contained within the handpiece are reservoirs (7) and (8) filled, respectively, with photosensitiser dye and sealant composition. Feed tubes (9) and (10) connect the reservoirs to the tube (6). The reservoirs may be squeezable pouches so that on applying pressure to the respective pouch, dye or fluid resin can be injected as required into the tube (6) and thence into the prepared cavity (2) or recess in the tooth. Preferably, the reservoirs, fibre tip and tube are disposable.

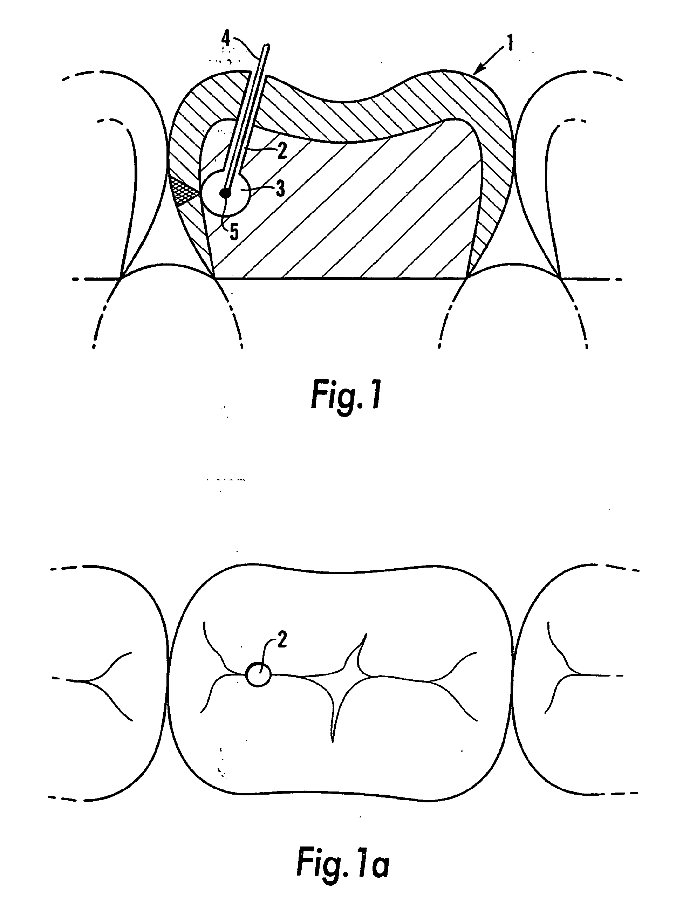

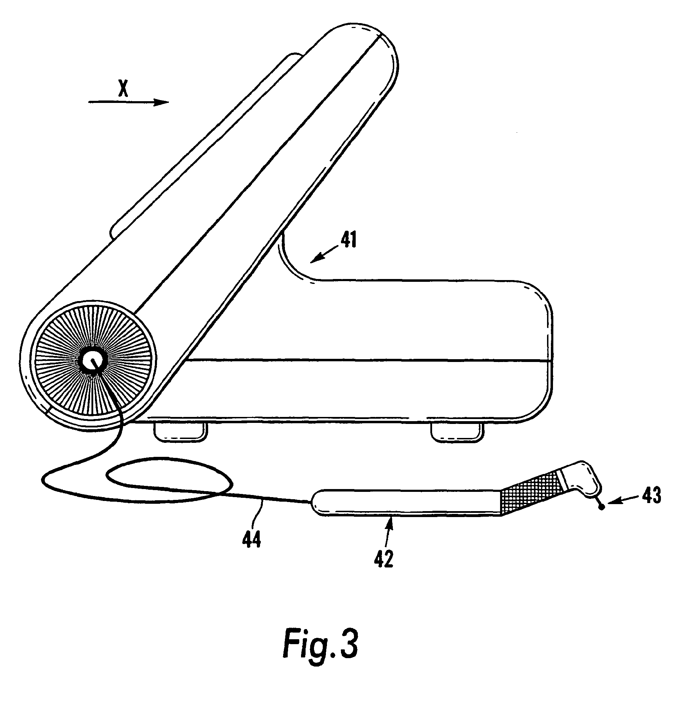

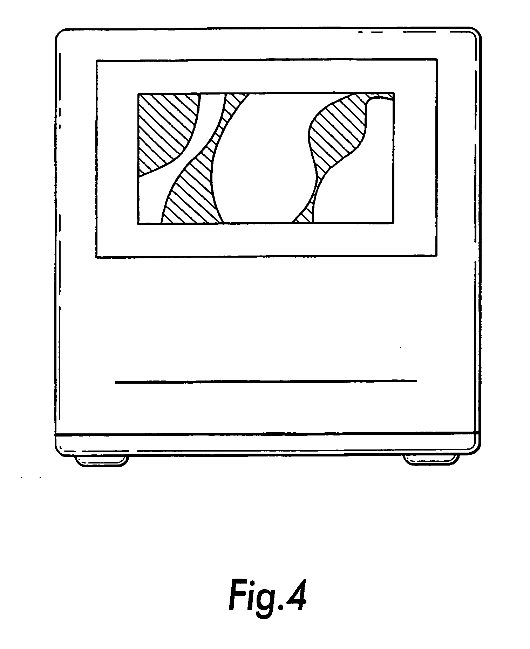

A more developed version of the laser console and a dental handpiece carrying the optical fibre is shown in Figures 3 and 4. Figure 3 shows a perspective view of the laser housing (41) linked to a dental handpiece (42). An optical fibre (43) is held in the part of the handpiece which will be introduced into the patient's mouth. The optical fibre (43) is a disposable "plug in" element which carries an isotropic tip as described above in relation to Figures 1 to 2. Housing (41) contains laser generating equipment whose output is connected to a flexible heavy duty optical fibre within the handpiece, the output from the fibre (44) is connected to the disposable fibre (43). Two laser sources may be accommodated in the housing (41), one capable of emitting laser light at a wavelength of about 670 nm for effecting the photosensitising treatment, and the other capable of emitting laser light for curing the resin sealant. Light guides and a beam splitter may be provided so that light from each laser source can be selectively switched to the tip (43). The fibre tip may be changed after the photosensitising treatment has been carried out and a fresh tip plugged in to the handpiece in order to carry out curing of the resin sealant.

Figure 4 shows a control panel (45) having a touch screen (46) for programming the laser power and duration of treatment. For convenience, the apparatus can be made in portable form and incorporate a rechargeable battery.

The following illustrates various ways in which the photodynamic therapy (PDT) system of the present invention may be applied to the treatment of different kinds of caries.
1. PDT with or without the novel resin may be used in a conventional cavity. Here, the cavity may be prepared with burs and hand instruments in the conventional manner. Once the soft bacteria infested caries has been removed, the cavity is filled with the solution of the dye, which is left for a period of time between 20 and 90 seconds, the preferred range is between 20 and 40 seconds. Excess dye solution is removed and the internal surfaces of the cavity exposed to light of the appropriate wavelength to activate the sensitiser for a period of time. The time of exposure will be between 20 and 90 seconds, and the preferred exposure time will be between 30 and 60 seconds. The power densities will range between 40 and 600 mW, c.g. from 40 to 150 mW, the preferred range being 40 to 80 mW. The delivery system for the light will consist of a fibre optic wand with preferably an isotropic emitter at the tip. This would provide a means of uniform radiation over the internal surface of the prepared cavity. A preferred method of treatment to enhance access to bacteria remaining within the mineralised part of the tooth and to ensure the photosensitising agent achieved maximum effect, would be to use solutions which open up the dentinal tubules. These would be used prior to application of the photosensitiser. Such solutions are those acids, demineralising agents and wetting agents as described above.
   The cavity may be restored with conventional restorative agents or, alternatively, the novel resin may be used as a base. This may be achieved by first applying the resin solution and then applying the resin in its filled form, since this will provide a mouldable paste. Preferably, light of an appropriate wavelength will be used to polymerise the resin after placement and provide a hermetic seal. Alternatively, auto-polymerisation may be carried out. The outer covering of the material may be with conventional restoratives.
2. PDT/Resin use in the treatment of caries.
   Here, the lesion is found at the cervical margin of the crown and on the root face. The lesion is generally extensive but relatively shallow. The lesion is initially treated with the photosensitiser. This may be in solution or gel form, and will be worked into the lesion using appropriate implements such as brushes or gel applicators. The time of exposure will be within the range of 20 to 90 seconds. The lesion is then exposed to light of the appropriate wavelength for times ranging from 30 to 90 seconds. The power densities used will be in the range of 40 to 600 mW, e.g. about 40 to 100 mW. To facilitate the exposure of the lesion to light, the isotropic tip may be passed through a strip with an internal reflective surface. This is adapted to the surface of the lesion and reflects light back onto the lesion surface. This matrix will be of a similar shape and configuration to the tooth surface. The uptake of dye by the lesion may again be enhanced by the use of solutions as described above. After exposure to the photosensitiser and light, a low viscosity resin will be painted over the surface and worked into the lesion. Preferably, light of an appropriate wavelength will be used to polymerise the resin after placement and provide a hermetic seal. Alternatively, auto-polymerisation may be carried out.
3. PDT/Resin as a fissure sealant.
   PDT may be used as an adjunct to conventional fissure sealing. Prior to use of the conventional resin, the fissures are acid etched with 35% phosphoric acid for 15 to 30 seconds. The photosensitiser is applied in solution form to the fissure system for a period of between 10 to 40 seconds. Excess solution is removed and the fissures irradiated with light of a suitable wavelength using a fibre optic wand with an isotropic tip at the end. The isotropic tip is drawn along the fissure system. After exposure, the fissures are coated with fissure sealant which is then polymerised using a visible light source of an appropriate wavelength.
4. PDT as an adjunct to convention advanced restorative work.
   It is well accepted that after any cavity or crown preparation, there are a substantial number of bacteria, which contaminate the dentine surface. The elimination of these prior to cementation of crowns and bridges reduces the risk of recurrent decay under the restoration. The use of PDT will reduce the risk by killing the bacteria on the surface of the preparation. The crown preparation is coated with the photosensitiser, which is left for a period of between 10 and 40 seconds. The preparation is then exposed to light of the appropriate wavelength to activate the photosensitiser. Exposure time is between 20 and 90 seconds, the preferred exposure time being between 20 and 60 seconds, with a power density of between 40 and 80 mW. The irradiation may be enhanced by placing a shroud with an internal surface of reflecting material over the preparation. The fibre optic delivery tip is placed through this and the reflective surface, used in conjunction with the isotropic tip, provides a uniform irradiation over the preparation surface. After exposure, the crown may be cemented in the normal way. Again, the uptake of photosensitiser may be enhanced by prior treatment of the preparation with the chemicals quoted earlier.
5. Treatment of recurrent caries beneath restorations.
   PDT and the novel resin may be used to treat recurrent decay beneath restorations. Once the lesion has been diagnosed, access is gained by cutting a tunnel cavity from the surface of the tooth to the lesion. This may be carried out using conventional instrumentation such as a small round bur (0.8mm diameter). The photosensitiser is injected down the tunnel preparation into the carious lesion. Exposure time is in the range of 20 to 90 seconds. Following exposure to the photosensitiser, the isotropic probe is introduced down the tunnel preparation. The site is irradiated with light of the appropriate wavelength for a range of times between 20 and 90 seconds. The exposure times are in the range of 20 to 60 seconds using a 40 to 150 mW power density. The uptake and penetration of the photosensitiser may be enhanced by the prior use of chemicals, which will demineralise the dentine and rehydrate the collagen The novel resin may then be syringed down the access tunnel and polymerised in situ, either by the use of light of the appropriate wavelength, or by auto-polymerisation.

## Claims

1. A dental carious lesion treatment kit which comprises:
(a) a flowable photosensitiser comprising a photosensitive dye,
(b) an optical fibre (4,44) which is shaped and dimensioned to enter a dental cavity or recess in which the lesion is located, said fibre being connectable proximally to laser light generating means for generating laser light at a wavelength which is strongly absorbed by the photosensitiser and
(c) a sealant or filling composition which is introducible into the cavity or recess and after curing forms an air-tight seal;
the photosensitive dye being effective to interact with the laser light to kill bacteria in the carious lesion,
**characterised by** said fibre having a distal portion (5) which is free from an internally light-reflecting layer and is shaped to spread emitted light around the walls of the recess or cavity

2. A kit as claimed in claim 1 wherein the sealant or filling composition has a low viscosity and is light curable.

3. A kit as claimed in claim 1 or claim 2 wherein the optical fibre (4,44) has a distal tip (5) so that it is capable of irradiating the region of the lesion in an arc of up to 360°.

4. A kit as claimed in claim 3 wherein the tip of the optical fibre has a lateral dimension of less than 1mm.

5. A kit as claimed in claim 3 wherein the tip of the optical fibre has a lateral dimension of 800 microns or less.

6. A kit as claimed in claim 3 wherein the tip of the optical fibre has a lateral dimension of 400 microns or less.

7. A kit as claimed in claim 3 wherein the tip of the optical fibre has a lateral dimension of about 200 microns.

8. A kit as claimed in any one of claims 1 to 7 further comprising materials selected from acids, wetting agents and demineralising agents for treating the cavity in order to open up dentine tubules prior to treating the lesion with a photosensitiser.

9. A kit as claimed in any one of claims 1 to 8 comprising a strip having a reflective surface and the tip of the optical fibre extending through said strip.

10. A kit as claimed in any one of claims 1 to 9 wherein the photosensitiser is an aqueous dye.

11. A kit as claimed in claim 10 in which the photosensitiser is an aqueous solution of toluidine blue O, aluminium disulphonated phthalocyanine chloride, methylene blue or azure blue chloride.

12. A kit as claimed in Claim 11 wherein the photosensitiser is an aqueous solution of toluidine blue O.

13. A kit as claimed in Claim 10 or Claim 11 wherein the concentration of the dye is in the range of 0.00001 % to 0.01%; preferably in the range of 0.001% to 0.01%.

14. A kit as claimed in any preceding claim further comprising a laser light generating means.

15. A kit as claimed in Claim 14 wherein the generated laser light has a power of from 20 to 600mW; preferably from 40 to 200mW; more preferably from 40 to 150mW.

## Patentansprüche

1. Kit zur Behandlung von kariösen Zahnläsionen, umfassend:
(a) einen fließfähigen Photosensibilisator, der einen lichtempfindlichen Farbstoff umfasst,
(b) eine optische Faser (4, 44), die so geformt und bemessen ist, dass sie in eine Pulpahöhle oder -vertiefung, in der die Läsion sich befindet, gelangt, wobei die Faser mit einem Mittel zur Erzeugung von Laserlicht zur Erzeugung von Laserlicht mit einer Wellenlänge, die vom Photosensibilisator stark absorbiert wird, proximal verbindbar ist und
(c) eine Dichtmittel- oder Füllmaterial-Zusammensetzung, die in die Höhle oder Vertiefung einführbar ist und nach dem Härten eine luftdichte Versiegelung bildet,
wobei der lichtempfindliche Farbstoff dahingehend wirksam ist, dass er mit dem Laserlicht so in Wechselwirkung tritt, dass Bakterien in der kariösen Läsion abgetötet werden,
**dadurch gekennzeichnet, dass** die Faser einen distalen Teil (5) hat, der frei von einer inneren lichtreflektierenden Schicht ist und so geformt ist, dass er emittiertes Licht um die Wandungen der Vertiefung oder der Höhle herum verteilt.

2. Kit nach Anspruch 1, wobei die Dichtmittel- oder Füllmaterial-Zusammensetzung eine niedrige Viskosität hat und durch Lichteinwirkung härtbar ist.

3. Kit nach Anspruch 1 oder Anspruch 2, wobei die optische Faser (4, 44) eine distale Spitze (5) hat, so dass sie zum Bestrahlen des Läsionsbereichs in einem Bogen von bis zu 360° fähig ist.

4. Kit nach Anspruch 3, wobei die Spitze der optischen Faser eine seitliche Abmessung von weniger als 1 mm hat.

5. Kit nach Anspruch 3, wobei die Spitze der optischen Faser eine seitliche Abmessung von 800 µm oder weniger hat.

6. Kit nach Anspruch 3, wobei die Spitze der optischen Faser eine seitliche Abmessung von 400 µm oder weniger hat.

7. Kit nach Anspruch 3, wobei die Spitze der optischen Faser eine seitliche Abmessung von etwa 200 µm hat.

8. Kit nach einem der Ansprüche 1 bis 7, weiterhin umfassend aus Säuren, Benetzungsmitteln und Demineralisierungsmittel ausgewählte Materialien zur Behandlung der Höhle, um vor der Behandlung der Läsion mit einem Photosensibilisator Zahnkanälchen zu öffnen.

9. Kit nach einem der Ansprüche 1 bis 8, umfassend einen Streifen mit einer reflektierenden Oberfläche und die Spitze der optischen Faser, die sich durch den Streifen erstreckt.

10. Kit nach einem der Ansprüche 1 bis 9, wobei der Photosensibilisator ein wässriger Farbstoff ist.

11. Kit nach Anspruch 10, wobei der Photosensibilisator eine wässrige Lösung von Toluidin Blau O, disulfoniertem Aluminiumphthalocyaninchlorid, Methylenblau oder Azurblauchlorid ist.

12. Kit nach Anspruch 11, wobei der Photosensibilisator eine wässrige Lösung von Toluidinblau O ist.

13. Kit nach Anspruch 10 oder Anspruch 11, wobei die Konzentration des Farbstoffs im Bereich von 0,00001 % bis 0,01 %, vorzugsweise im Bereich von 0,001 % bis 0,01 % liegt.

14. Kit nach einem beliebigen der vorhergehenden Ansprüche, weiterhin umfassend ein Mittel zur Erzeugung von Laserlicht.

15. Kit nach Anspruch 14, wobei das erzeugte Laserlicht eine Leistung von 20 bis 600 mW, vorzugsweise von 40 bis 200 mW, noch mehr bevorzugt von 40 bis 150 mW hat.

## Revendications

1. Kit de traitement de caries dentaires qui comprend :
(a) un photosensibilisateur liquide comprenant un colorant photosensible,
(b) une fibre optique (4, 44) qui est conçue et dimensionnée afin de pénétrer dans une cavité ou un renfoncement dentaire dans laquelle/lequel la lésion est située, ladite fibre pouvant être reliée de manière proximale à un moyen de génération de lumière laser destiné à générer une lumière laser à une longueur d'onde qui est fortement absorbée par le photosensibilisateur, et
(c) un produit d'étanchéité ou une solution de remplissage qui peut être introduit(e) dans la cavité ou le renfoncement et, après durcissement, forme un joint étanche à l'air ;
le colorant photosensible étant efficace afin d'interagir avec la lumière laser de façon à tuer les bactéries dans la lésion cariée,
**caractérisé par** ladite fibre possédant une partie distale (5) qui est distincte d'une couche de réfléchissement interne de la lumière et qui est conçue afin de diffuser la lumière émise autour des parois du renfoncement ou de la cavité.

2. Kit selon la revendication 1, dans lequel le produit d'étanchéité ou la composition de remplissage possède une faible viscosité et est durcissable à la lumière.

3. Kit selon la revendication 1 ou la revendication 2, dans lequel la fibre optique (4, 44) possède une pointe distale (5) de telle sorte qu'elle soit capable d'irradier la région de la lésion selon un arc allant jusqu'à 360°.

4. Kit selon la revendication 3, dans lequel la pointe de la fibre optique possède une dimension latérale de moins de 1 mm.

5. Kit selon la revendication 3, dans lequel la pointe de la fibre optique possède une dimension latérale de 800 microns ou moins.

6. Kit selon la revendication 3, dans lequel la pointe de la fibre optique possède une dimension latérale de 400 microns ou moins.

7. Kit selon la revendication 3, dans lequel la pointe de la fibre optique possède une dimension latérale d'environ 200 microns.

8. Kit selon l'une quelconque des revendications 1 à 7, comprenant en outre des matériaux choisis parmi des acides, des agents humidificateurs et des agents déminéralisants afin de traiter la cavité de façon à ouvrir des tubules de dentine avant de traiter la lésion avec un photosensibilisateur.

9. Kit selon l'une quelconque des revendications 1 à 8, comprenant une bande possédant une surface réfléchissante et la pointe de la fibre optique s'étendant dans ladite bande.

10. Kit selon l'une quelconque des revendications 1 à 9, dans lequel le photosensibilisateur est un colorant aqueux.

11. Kit selon la revendication 10, dans lequel le photosensibilisateur est une solution aqueuse de bleu de toluidine O, de chlorure de phthalocyanine bisulfuré d'aluminium, de bleu de méthylène ou de chlorure de bleu azur.

12. Kit selon la revendication 11, dans lequel le photosensibilisateur est une solution aqueuse de bleu de toluidine O.

13. Kit selon la revendication 10 ou la revendication 11, dans lequel la concentration de colorant est de l'ordre de 0,00001% à 0,01% ; de préférence de l'ordre de 0,001% à 0,01%.

14. Kit selon l'une quelconque des revendications précédentes, comprenant en outre un moyen de génération de lumière laser.

15. Kit selon la revendication 14, dans lequel la lumière laser générée possède une puissance de 20 à 600 mW ; de préférence de 40 à 200 mW ; de préférence de 40 à 150 mW.
